# EUROPÄISCHE PATENTSCHRIFT

(11) **EP 0 355 688 B1**
(45) Veröffentlichungstag und Bekanntmachung des Hinweises auf die Patenterteilung: **13.04.1994**
(21) Anmeldenummer: 89115091.4
(22) Anmeldetag: 16.08.1989
(51) Int. Cl.: G01N 33/32

(54) **Verfahren zur Messung des Alkoholgehaltes der Feuchtflüssigkeit für Alkohol-Feuchtwerke von Offset-Druckmaschinen**
Method for measuring the alcohol content of a damping liquid in an alcohol damping device of an offset printing machine
Procédé pour mesurer le taux d'alcool du liquide de mouillage pour un système de mouillage à l'alcool d'une machine d'imprimerie offset

(30) Priorität: 20.08.1988 DE 3828325
(43) Veröffentlichungstag der Anmeldung: 28.02.1990
(73) Patentinhaber: MAN Roland Druckmaschinen AG, 86153 Augsburg (DE)
(72) Erfinder: Rodriguez Giles, Jorge M., Dr., D-6200 Wiesbaden (DE)
(74) Vertreter: Grussdorf, Jürgen, Dr.

(56) Entgegenhaltungen:
- DE-A- 3 309 458
- ANALYTICAL CHEMISTRY, Band 42, Nr. 14, Dezember 1970; M. SUZUKI et al., Seiten 1705-1708#
- PATENT ABSTRACTS OF JAPAN, Band 7, Nr. 38 (P-176)[1183], 16. Februar 1983#
- PATENT ABSTRACTS OF JAPAN, Band 6, Nr. 166 (P-138)[1044], 31. August 1982#

## Beschreibung

Die Erfindung betrifft ein Verfahren zur Messung des Alkoholgehaltes der Feuchtflüssigkeit für Alkohol-Feuchtwerke von Offset-Druckmaschinen. Eine solche Messung ist erforderlich zwecks Konstanthaltung des Alkoholgehaltes auf seinem Sollwert. Dies erfolgt durch mehr oder weniger starkes Zuimpfen von Alkohol zur Feuchtflüssigkeit zusätzlich zu der üblichen konstanten, auf Erfahrungswerten beruhenden Alkoholzuführung oder aber auch einer von bestimmten Parametern, ebenfalls nach Erfahrungswerten, abhängigen Alkoholzuführung. Dieses Zuimpfen bewirkt also nur noch eine Feineinstellung des Sollwertes, während die Grobeinstellung durch die konstante oder parameterabhängige Alkoholzuführung erfolgt.

Die Messung des Alkoholgehaltes im Feuchtmittel von Alkohol-Feuchtwerken von Offset-Druckmaschinen erfolgt bisher durch Messung der Dichte des Feuchtmittels, also durch Ausspindeln oder Aerometer. Diese Meßart ist aber recht ungenau. Sie berücksichtigt nämlich nicht die Tatsache, daß das spezifische Gewicht (die Dichte) des Feuchtmittels nicht allein vom Alkoholgehalt abhängt, (meistens wird Isopropylalkohol, d.h.also 2-Propanol verwendet), sondern auch, und zwar in ziemlich starkem Maße, von der Menge an Zusatzstoffen zwecks Einhaltung eines bestimmten pH-Wertes, der etwa im Bereich zwischen 4 bis 6,5 liegen soll. Außerdem werden Feuchtmittelzusätze für den Plattenschutz, oberflächenaktive Substanzen, antimikrobielle Wirkstoffe, Komplexbildner zur Kompensation hoher Wasserhärten, antikorrosiv wirkende Substanzen und anderes mehr zugesetzt. Alle diese Stoffe beeinflussen das spezifische Gewicht in einer solchen Weise,daß der durch Dichtemessung ermittelte Alkoholgehalt oft 50 bis 100% höher gemessen wird, als er tatsächlich durch eine chemische Analyse ermittelt wird. Zu hohe Alkoholgehalte bedeuten im übrigen nicht nur eine Verschwendung des einigermaßen kostspieligen Alkohols, sondern verstärkte Immission, Anlösung der Farbe und überhaupt eine nicht optimale Qualität der Druckarbeit. Schon aus diesem Grunde ist man bestrebt, mit möglichst geringen Alkoholgehalten zu arbeiten, etwa in der Größenordnung von 6%, aber gerade dann ist die sehr genaue Einhaltung des Sollwertes von äußerster Wichtigkeit, weil ja eine Abweichung von einem Prozent Alkoholanteil in diesem Falle viel mehr ausmacht, als wenn der Sollwert beispielsweise 15% betragen würde.

In der DE 33 09 458A1, Patent Abstract of Japan, Band 7, Nr. 38 (P-176) (1183), 16. Februar 1983 = JP-A 57 191 542, Patent Abstract of Japan, Band 6, Nr. 166 (P 138) (1044), 31. August 1982 = JP-A-5782758; sowie Analytical Chemistry, Band 42 Nr. 14, Dezember 1970, Seiten 1705-1708, sind Meßmethoden für die Zusammensetzung oder Bestimmung von Komponenten von Basen angegeben. Diese Literaturstellen offenbaren jedoch keine Möglichkeit, die Qualität von Druckerzeugnissen durch Regelung des Alkoholgehaltes in der Feuchtflüssigkeit von Offset Druckmaschinen zu beeinflussen.

Aufgabe der Erfindung ist daher die Schaffung eines ganz wesentlich genauer arbeitenden Verfahrens für die Messung des Alkoholgehaltes, das aber mit einfachen und daher nicht aufwendigen Mitteln und Geräten zu verwirklichen ist.

Diese Aufgabe wird nach der Erfindung in der im Hauptanspruch wiedergegebenen Weise gelöst. Vorteilhafte Ausgestaltungen des Verfahrens finden sich in den Unteransprüchen.

Die erfindungsgemäße Lösung beruht auf der Tatsache, daß die in dem Feuchtmittel nach oben steigenden Luftbläschen, wenn sie den Feuchtmittelspiegel erreicht haben, einen bestimmten Wasserdampf- und Alkohol-Sättigungsdruck haben, und der Sättigungsdampfdruck von Alkohol bei einer konstanten Temperatur direkt proportional zur Alkoholkonzentration ist, was auf bekannten Gesetzen der physikalischen Chemie beruht. Solche Geräte zur Messung des Gehaltes eines Gas-Dampf-Gemisches an brennbarem Gas oder Dampf sind in vielfacher Form bekannt, beispielsweise in und auf Tankern, Bohrinseln, Gruben (zwecks Feststellung von Methangasexplosionsgefahr) und auf noch vielen weiteren Anwendungsgebieten. Die Konstanthaltung der Feuchtmitteltemperatur bringt ohnehin keine zusätzlichen Probleme mit sich, da das Feuchtmittel ja ohnehin in dem Feuchtmittel-Aufbereitungsgerät auf eine bestimmte, konstante Temperatur rückgekühlt wird. Die zwecks Grobeinstellung zugeführte, konstante oder von bestimmten Parametern abhängige Alkoholmenge richtet sich, wie schon immer, unter anderem nach dem Maschinentyp, der Druckgeschwindigkeit, dem Sujet, dem Druckplattentyp, dem Bedruckstoff und der sich auf die verdunstende Alkoholmenge auswirkenden Außentemperatur.

Die Mittel zur Durchführung des Verfahrens nach der Erfindung sind also, wie gefordert, sehr einfach, und die zur Verwirklichung der Erfindung erforderlichen Meßgeräte stehen bereits in vielfacher Auswahl und in erprobten Ausführungen zur Verfügung.

Das Verfahren nach der Erfindung kann auch in der Weise verwirklicht werden, daß zwar die Temperatur der Flüssigkeit nicht konstant gehalten wird, jedoch diese Temperatur gemessen und bei der Ermittlung der Alkoholkonzentration in der Flüssigkeit berücksichtigt wird.

Die Bildung eines Gas-Dampf-Gemisches kann auch in einer Tropfkammer (auch Wäscher genannt) erfolgen. Ferner ist es auch möglich, daß die Bildung des Gas-Dampf-Gemisches durch Verdunstung an einer freien Flüssigkeitsoberfläche, an einer mit der Flüssigkeit benetzten oder sonstigen anderen Flüssigkeitsoberfläche erfolgt. Weiterhin ist es auch möglich, daß die Bildung des Gas-Dampf-Gemisches durch Verdunstung durch eine Membrane erfolgt.

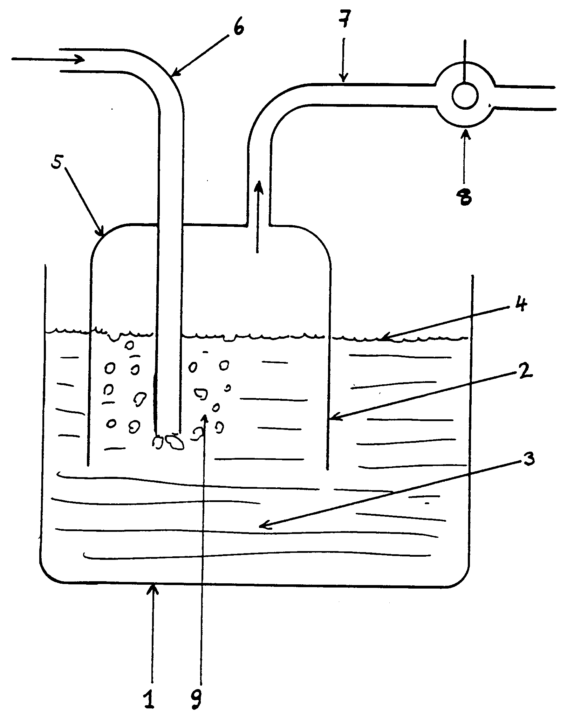

In der Zeichnung ist ganz schematisch eine Art einer Vorrichtung gezeigt, die zur Durchführung des Verfahrens nach der Erfindung dient.

In einem Behälter 1 beliebiger Art, der z.B. ein großer Behälter in einem zentralen Feuchtmittel-Aufbereitungsgerät für eine Vielzahl von Druckmaschinen sein kann, aber auch der Feuchtmittelkasten eines Feuchtwerkes, befindet sich die Feuchtflüssigkeit 3. In diesen Behälter 1 taucht ein glockenartiger Behälter 2 ein, der oben durch eine Abdeckung 5 verschlossen wird. In das Innere dieses Innenbehälters 2 wird über eine Leitung 6 Druckluft eingeblasen, wodurch in diesem inneren Behälter 2 Bläschen 9 aufsteigen, die, wenn sie den Feuchtmittelspiegel 4 erreichen und durchbrechen, eine Wasserdampf-Alkoholdampf-Konzentration enthalten, die genau der Alkoholkonzentration der Feuchtflüssigkeit 3 entspricht. Über eine Leitung 7 wird ein Teil dieses Gemisches einem Meßgerät 8 zugeleitet, welches den Gehalt dieses Gemisches an brennbarem Gas, bei dem es sich nur um Alkoholdampf handeln kann, und damit die Alkoholkonzentration der Feuchtflussigkeit 3 mißt.

## Patentansprüche

1. Verfahren zur Feineinstellung der Zusammensetzung der Zusatzstoffe enthaltenden Feuchtflüssigkeit von Offsetdruckmaschinen durch Bestimmung von deren Alkoholgehalt, **dadurch gekennzeichnet**, daß man zur Vermeidung von Überdosiermengen in oder auf die in einem Feuchtmittelbehälter befindliche Flüssigkeit ein Gas leitet, dieses mit dem Feuchtmittel oder Feuchtmitteldampf vermischt, dabei im Gas Alkohol löst, die im Gas aufgenommene Alkoholmenge auf bekannte Weise ermittelt und die Konstanthaltung des Alkoholgehaltes durch, zu der üblichen, konstanten von Erfahrungswerten abhängigen Alkoholzuführung zusätzliches Zuimpfen von Alkohol zum Feuchtmittel nach dem ermittelten Wert regelt.

2. Verfahren nach Anspruch 1, **dadurch gekennzeichnet**, daß man die Feuchtflüssigkeit auf konstanter Temperatur hält.

3. Verfahren nach Anspruch 1, **dadurch gekennzeichnet**, daß bei nicht konstant gehaltener Temperatur die Temperatur der Feuchtflüssigkeit festgestellt und bei der Ermittlung der Alkoholkonzentration im Dampf/Gasgemisch berücksichtigt wird.

4. Verfahren nach einem der Ansprüche 1 - 3, **dadurch gekennzeichnet**, daß die Bildung des Gas/Dampfgemisches in einer Tropfkammer erfolgt.

5. Verfahren nach einem der Ansprüche 1 - 3, **dadurch gekennzeichnet**, daß man den Feuchtflüssigkeitsdampf durch Verdunstung an einer Membrane erzeugt.

## Claims

1. Process for the fine adjustment of the composition of the damping liquid of offset printing machines containing additives by determination of its alcohol content, characterised in that, for the avoidance of overdosed amounts, into or on to the liquid present in the damping agent container one passes a gas, mixes this with the damping agent or damping agent vapour, thereby dissolves alcohol in the gas, determines in known manner the amount of alcohol taken up in the gas and regulates the keeping constant of the alcohol content according to the determined value by introduction of alcohol to the damping agent in addition to the alcohol introduction dependent upon the usual constant values known from experience.

2. Process according to claim 1, characterised in that one keeps the damping liquid at constant temperature.

3. Process according to claim 1, characterised in that, in the case of temperature not kept constant, the temperature of the damping liquid is ascertained and this is taken into account in the case of the determination of the alcohol concentration in the vapour/gas mixture.

4. Process according to one of claims 1 - 3, characterised in that the formation of the gas/vapour mixture takes place in a dropping chamber.

5. Process according to one of claims 1 - 3, characterised in that one produces the damping liquid vapour by evaporation on a mambrane.

## Revendications

1. Procédé de réglage fin de la composition du liquide mouillant, contenant des additifs, pour des machines d'imprimerie offset par détermination de sa teneur en alcool, caractérisé en ce que, pour éviter des quantités de dosage excessives, on amène un gaz dans ou sur le liquide qui se trouve dans un récipient de milieu mouillant, celui-ci se mélange au milieu mouillant ou à de la vapeur du milieu mouillant, alors de l'alcool se dilue dans le gaz, la quantité d'alcool prise dans le gaz est déterminée d'une manière connue et on règle suivant la valeur déterminée la constance de la teneur en alcool par injection d'alcool, supplémentaire à l'alimentation en alcool usuelle constante et dépendant de valeurs d'expérience, dans le milieu mouillant.

2. Procédé suivant la revendication 1, caractérisé en ce qu'on maintient le liquide mouillant à une température constante.

3. Procédé suivant la revendication 1, caractérisé en ce que, lorsque la température n'est pas maintenue constante, la température du liquide mouillant est déterminée et est prise en considération lors de la détermination de la concentration en alcool dans le mélange de gaz et de vapeur.

4. Procédé suivant l'une quelconque des revendications 1 à 3, caractérisé en ce que la formation du mélange de gaz et de vapeur a lieu dans une chambre d'égouttage.

5. Procédé suivant l'une quelconque des revendications 1 à 3, caractérisé en ce qu'on produit la vapeur de liquide mouillant par évaporation sur une membrane.
